# EUROPÄISCHE PATENTSCHRIFT

(11) **EP 1 393 741 B1**
(45) Veröffentlichungstag und Bekanntmachung des Hinweises auf die Patenterteilung: **05.05.2010**
(21) Anmeldenummer: 03016766.2
(22) Anmeldetag: 23.07.2003
(51) Int. Cl.: A61K 38/17, A61P 7/00

(54) **Lagerungsstabile, flüssige Fibrinogen-Formulierung**
Storage-stable, liquid fibrinogen-formulation
Formulation du fibrinogene stable et liquid

(30) Priorität: 13.08.2002 DE 10237643; 20.12.2002 DE 10261126
(43) Veröffentlichungstag der Anmeldung: 03.03.2004
(73) Patentinhaber: CSL Behring GmbH, 35041 Marburg (DE)
(72) Erfinder: Metzner, Hubert, 35041 Marburg (DE); Liebing, Uwe, 35091 Coelbe (DE); Kumpe, Gerhardt, 35083 Wetter (DE); Schulte, Stefan, 35043 Marburg (DE); Gawantka, Volker, 35094 Lahntal (DE); Enssle, Karlheinz, 39041 Marburg (DE)

(56) Entgegenhaltungen:
- EP-A- 0 804 933
- DE-A- 10 012 732
- US-B1- 6 277 961
- JANSSEN C L ET AL: "Conditions for stabilization and determination of activated factor V" THROMBOSIS RESEARCH 1974, Bd. 5, Nr. 3, 1974, Seiten 315-325, XP009016168
- TAKEBE MIKIHIRO ET AL: "Calcium ion-dependent monoclonal antibody against human fibrinogen: Preparation, characterization, and application to fibrinogen purification." THROMBOSIS AND HAEMOSTASIS, Bd. 73, Nr. 4, 1995, Seiten 662-667, XP009016167 ISSN: 0340-6245

## Beschreibung

Die vorliegende Erfindung betrifft eine neue lagerungsstabile Formulierung für Fibrinogen in flüssiger oder viskos flüssiger Form enthaltend zweiwertige Metallionen und einen Komplexbildner. Die Fibrinogenformulierung kann weitere übliche Formulierungsbestandteile enthalten. Weiterhin betrifft die Erfindung die Verwendung der erfindungsgemäßen Fibrinogenformulierungen.

Fibrinogen ist ein Protein das vorwiegend in der Leber gebildet wird und etwa 2-3% des Proteingehalts des Blutplasmas ausmacht. Es spielt eine Schlüsselrolle bei der Blutgerinnung. Bei Verletzungen oder Operationen werden fast immer Blutgefäße beschädigt und es entstehen Blutungen. Durch die Blutgerinnung wird das Blut im Bereich kleinerer Wunden verfestigt und die Blutung kommt zum Stillstand. Das Gerinnungssystem schützt den Körper damit vor hohen Blutverlusten. Bei der Blutgerinnung wird das im Blutplasma enthaltene lösliche Fibrinogen in das faserartige unlösliche Fibrin umgewandelt und verhilft dem Thrombus so zu seiner endgültigen Stabilität. Die Umwandlung von Fibrinogen in Fibrin erfolgt in Anwesenheit von Thrombin. Thrombin wird bei Verletzungen über ein kompliziertes Kettenreaktionssystem, an dem viele verschiedene Gerinnungsfaktoren beteiligt sind, im Blut freigesetzt.

Wegen seiner Bedeutung für die Blutstillung und Wundheilung hat Fibrinogen eine hohe Bedeutung in der klinischen Anwendung.

Fehlt es an Fibrinogen, so funktioniert die Blutgerinnung nicht korrekt. Man kann den Mangel ausgleichen, indem man Fibrinogen, z.B. isoliert aus menschlichem Blutplasma, verabreicht. Ein Mangel an Fibrinogen kann z.B. durch großflächige Wunden (z. B. schwere Verbrennungen), disseminierte, intravasale Gerinnung oder starke Blutungen verursacht sein. Hohe Bedeutung kommt dem Fibrinogen auch als Komponente eines Fibrinklebers zu, bei dem Fibrinogen in der Regel durch Zusatz einer calciumhaltigen Thrombinlösung in Fibrin umgewandelt wird. Eingesetzt wird ein solcher Kleber beispielsweise zum Sichern oder zum Abdichten von Nähten, vorzugsweise bei chirurgischen Eingriffen. Besonders auch an Weichteilorganen wie Leber und Milz lässt er sich vorteilhaft zur Erzielung der Blutstillung oder Abdichtung einsetzen.

Die Stabilität von Proteinen ist in der pharmazeutischen Industrie ein generelles Problem, das für jedes Protein im Detail neu zu lösen ist. In Abhängigkeit vom Protein können einzelne Formulierungsbestandteile einen großen Einfluss auf die Stabilität haben, wobei die Formulierungsbestandteile auch abhängig von der geplanten Lagerungsform und Lagerungstemperatur sein können.

Häufig werden Proteine unter Zusatz bestimmter Hilfsstoffe lyophilisiert und in trockener Form gelagert. Dabei muss ein Stabilitätsverlust während der Trocknung möglichst vermieden werden und auch bei der Rekonstitution sollte es zu keinem Wirkungsverlust kommen. Mögliche Probleme bei der Rekonstitution sind z.B. die Bildung von Flocken bzw. Trübungen oder lange Zeiträume bis zum völligen Lösen des Proteins, insbesondere bei hohen Proteinkonzentrationen. Es ist daher von Vorteil, wenn dieser Schritt vermieden werden kann. Das häufig alternativ verwendete Einfrieren von Proteinproben hat den Nachteil, dass das Auftauen und Erwärmen Zeit benötigt, Lagertemperaturen von unter 0°C gewährleistet sein müssen und mehrfaches Frieren und Tauen zumeist mit Wirkungsverlusten verbunden ist.

Ein großer Vorteil ist bei einer Aufbewahrung in flüssiger Form demnach generell und insbesondere auch für die Fibrinogen-Formulierung die sofortige Einsatzmöglichkeit des Wirkstoffes am Patienten, da sich der zeitliche Aufwand der Rekonstitution lyophilisierter Formulierungen bzw. das Auftauen und Erwärmen eingefrorener Formulierungen erübrigt. Aber auch bei einer zwischenzeitlichen Lagerung als Lyophilisat oder im gefrorenen Zustand ist es von Vorteil, wenn die rekonstituierte bzw. aufgetaute, dann in flüssiger Form vorliegende Fibrinogen-Formulierung, noch länger stabil ist. Dies zeigt sich beispielsweise in Situationen, wo z.B. für Operationen vorsorglich Material rekonstituiert wurde, dann aber aus medizinischen Erwägungen heraus der Einsatz nicht notwendig wurde. Dieses Material müsste bei nur kurzfristiger Stabilität verworfen werden und könnte nicht mehr zu einem späteren Zeitpunkt zum Einsatz kommen. Ein stabilitätsbedingter Verlust an Material ist aber insbesondere zu vermeiden, wenn es sich um therapeutische Substanzen handelt, die von menschlichen Spenden herrühren, da diese nur in begrenzten Mengen verfügbar sind. Fibrinogen zählt zu diesen Substanzen, da es vorwiegend aus menschlichem Blutplasma gewonnen wird.

Bei Verwendung von Fibrinklebern ist es insbesondere von Vorteil, wenn Fibrinogen in flüssiger Form vorliegt. Bei den kommerziell erhältlichen Klebern handelt es sich zumeist um zwei Komponenten. Die eine Komponente umfasst Fibrinogen, häufig zusammen mit Faktor XIII und Aprotinin, und die andere Komponente umfasst Thrombin, häufig zusammen mit Calciumionen. Das Rekonstituieren, damit der Kleber einsatzbereit ist, benötigt relativ viel Zeit, zumal Fibrinogen in hohen Konzentrationen vorliegt. Ein vorsorgliches Bereitstellen des Klebers beinhaltet aber den Nachteil, dass die Effizienz des Klebers nachlässt oder er sogar insgesamt unbrauchbar wird, selbst wenn die thrombinhaltige Lösung stabil bleiben sollte.

Ein weiterer großer Vorteil der erfindungsgemäßen Fibrinogen-Formulierung in flüssiger oder viskos-flüssiger Form ist die Alterungsbeständigkeit, wodurch die Möglichkeit einer Lagerung für eine bestimmte Zeit auch bei Raumtemperatur und damit die Anwendungseigenschaften in Notfallsituationen verbessert werden können. Auch auf längeren Versandwegen, wo tiefe Temperaturen nicht durchgehend gewährleistet werden können, ist es von Vorteil, wenn die Stabilität auch bei Raumtemperatur über einen längeren Zeitraum zu gewährleisten ist. In seltenen Fällen, wo ein chronischer Fibrinogenmangel vorliegt (z.B. bei ererbten Störungen der Fibrinogensynthese), kann es auch von Vorteil sein, wenn Fibrinogen dem Patienten kontinuierlich zugeführt werden kann, was ein körpernahes Tragen der Fibrinogenlösung, entsprechend einer Lagertemperatur von ca. 30 °C, voraussetzt.
Stabile Flüssigformulierungen von Fibrinogen erleichtern also in vielerlei Hinsicht die Herstellung, den Gebrauch, Transport und die Verabreichung am Patienten.

Es gibt Veröffentlichungen zu Plasmaproteinen/Gerinnungsfaktoren, in denen gezeigt wird, dass neben anderen Bestandteilen auch Calcium in Flüssigformulierungen verwendbar ist. Verwiesen sei hierbei beispielsweise auf die WO 96/30041 und die US 5,925,738, wo Stabilitätsuntersuchungen an Flüssigformulierungen von Faktor VIII und/oder FIX durchgeführt wurden. Verminderte Aktivitäten von Faktor VIII bzw. FIX verursachen Hämophilie A bzw. B. Für Faktor VIII ist z.B. die Assoziation der schweren und leichten Kette abhängig von zweiwertigen lonen wie Calcium. Daten, die für diese Faktoren gewonnen wurden, sind jedoch nicht naheliegend übertragbar auf das strukturell und funktionell völlig verschiedene Fibrinogen. Bei Fibrinogen handelt es sich um ein sehr großes Protein mit einer komplizierten Struktur. Es ist ein aus zwei symmetrischen Hälften bestehendes Glykoprotein von ca. 340 kDa. Je zwei alpha, beta und gamma-Ketten liegen in einer länglichen Form (ca. 47 nm) vor, die drei Domänen bildet (eine zentrale E Domäne und zwei identische D Domänen). Diese komplizierte Struktur ist unverzichtbar für die Bildung von Fibrin. Fibrinogen ist als Substrat die Vorstufe für Fibrin, das eine strukturelle Matrix bildet. Im Gegensatz dazu sind Gerinnungsfaktoren wie beispielsweise Faktor VIII und Faktor IX in ihrer aktivierten Form Enzyme oder Cofaktoren, die ihrerseits weitere, an der Kettenreaktion zur Bildung von Thrombin beteiligte Faktoren (FX), aktivieren oder deren Aktivierung beschleunigen. Bei einer Lagerung von Fibrinogen muss nicht nur gewährleistet sein, dass die komplizierte Struktur erhalten bleibt, sondern zudem, dass sich Fibrinogen nicht zu kovalent vernetzten, fibrinähnlichen Strukturen zusammenlagert. Außerdem wird im Stand der Technik bei längeren Lagerungen von Fibrinogenkonzentraten im flüssigen Zustand möglichst vermieden, Fibrinogen mit Calcium zu kombinieren. Dies wird ersichtlich z.B. bei den kommerziell erhältlichen Fibrinklebern. Hierbei wird das für die Bildung von quervernetztem, stabilen Fibrin notwendige Calcium zur Lagerung der Thrombinkomponente zugesetzt oder von beiden Komponenten getrennt gehalten, aber nicht mit der Fibrinogenkomponente kombiniert. Im Falle eines Einkomponentenklebers mit den Bestandteilen Fibrinogen, FXIII, Prothrombinfaktoren, eines Thrombininhibitors und Plasmininhibitors, der aus praktischen Handhabungsüberlegungen in der EP 0 253 198 entwickelt wurde, wird die optimale Calciumionenkonzentration nur in Hinblick auf die Aktivierung von Thrombin eingestellt und liegt in der Anwendungslösung bei nur 0,5-1 mM. Es wird nirgends erwähnt, dass Calciumionen einen stabilisierenden Effekt gegen eine nicht-proteolytische Inaktivierung von Fibrinogen ausüben. Zudem liegt das Gemisch der Wirkstoffe für den Kleber vorzugsweise in fester Form, wie bevorzugt als Lyophilisat, vor. Die hier beschriebenen und beanspruchten Fibrinogenformulierungen enthalten aber keine signifikanten Mengen an Prothrombinfaktoren oder Thrombin.

Ein Vermeiden der gleichzeitigen längerfristigen Lagerung von Fibrinogen und Calciumionen im flüssigen Zustand ist auch aus den Fällen ersichtlich, wo humanes Plasma oder Fraktionen davon, die für die Gewinnung von Fibrinogen verwendet werden, für die Hitzebehandlung (Pasteurisierung) u.a. mit Calciumionen versetzt werden. Durch die nachfolgenden Prozessschritte wie Diafiltration oder Fällung wird Calcium wieder abgereichert, so dass die endgültige Fibrinogen-Formulierung praktisch ohne nennenswerte Mengen an Calciumionen vorliegt, d.h. bei etwa 1 mMol/l oder weniger. Verwiesen sei hier auf die EP 0 103 196, in der während des Pasteurisierungsschrittes 5 mM CaCl₂ verwendet wird, das dann aber sowohl in der Formulierung für die intravenöse Infusion, als auch in der Formulierung für die Herstellung von Fibrinklebern wieder abgereichert wird. Lösungsbestandteile, die für eine Behandlung bei hoher Temperatur über einen kurzen Zeitraum (Pasteurisierung) verwendet werden, sind natürlich auch nicht automatisch verwendbar in Lösungen, die bei niedrigen Temperaturen oder Raumtemperatur für mehrere Monate oder sogar Jahre gelagert werden sollen.

Schließlich ist auch aus der Patentanmeldung WO 01/48016 bereits ein Verfahren zur Reinigung von Fibrinogen bekannt, bei dem aus dem Fraktion I-Präzipitat Fibrinogen mit einem Extraktionspuffer herausgelöst wird, der Calciumionen enthalten kann. Bei den beschriebenen Verfahrensschritten werden Plasminogen-Verunreinigungen beseitigt, die eine proteolytische Inaktivierung von Fibrinogen zur Folge haben können. Über eine Stabilisierung von Fibrinogen durch zweiwertige Metallionen gegenüber einer nicht-proteolytischen Inaktivierung bei Lagerung ist bisher noch nicht berichtet worden.

Mit der vorliegenden Erfindung konnte überraschenderweise gezeigt werden, dass bei längeren Lagerungen in flüssiger Form im Temperaturbereich von 0-30°C die nicht-proteolytische Inaktivierung von Fibrinogen durch Zusatz zweiwertiger Metallionen verhindert oder reduziert werden kann. Weitere Steigerungen der Stabilität konnten überraschenderweise durch gleichzeitige Verwendung von Komplexbildnern erreicht werden. Das erfindungsgemäße Fibrinogenpräparat ist im flüssigen Zustand stabil und dabei weitgehend frei von kovalenten Fibrinogen-Aggregaten mit mehr als fünf Fibrinogenmolekülen.

Die der vorliegenden Erfindung zugrunde liegende Aufgabe besteht also darin, eine lagerungsstabile Fibrinogen-Formulierung bereitzustellen, die im flüssigen oder viskos-flüssigen Zustand bei Lagerung im Temperaturbereich von 0-30°C, bevorzugt bei 2-8°C, mindestens einen Monat stabil ist. Diese Aufgabe wird gelöst, durch eine Formulierung, die neben Fibrinogen zweiwertige Metallionen in einer Konzentration von bis zu 100 mM und einen Komplexbildner sowie gegebenenfalls ein oder mehrere weitere Formulierungsbestandteile enthält. Weitere Ausführungsformen betreffen den Gegenstand der Ansprüche 2 bis 24 und weitere Merkmale und Vorteile der Erfindung gehen aus der Beschreibung der bevorzugten Ausführungsformen und den Beispielen hervor.

Die vorliegende Erfindung umfasst eine Formulierung für Fibrinogen (Fibrinogen-Formulierung), die durch den Zusatz von zweiwertigen Metallionen und einem Komplexbildner sowie gegebenenfalls ein oder mehreren weiteren Formulierungsbestandteilen im flüssigen oder viskos-flüssigen Zustand im Temperaturbereich von 0-30°C, bevorzugt bei 2-8°C, mindestens einen Monat stabil ist. Der Begriff "stabil" im Sinne der vorliegenden Erfindung bedeutet, dass bei der Lagerung der Fibrinogen-Formulierung kein wesentlicher Wirkungsverlust durch nicht-proteolytische Inaktivierung auftritt, zumindest aber 70% der Ausgangsaktivität erhalten bleibt. Der Wirkungsverlust ist dabei bevorzugt nach der Methode zur Feststellung von gerinnbarem Protein, wie beschrieben in der Fibrinkleber-Monographie des Europäischen Arzneibuches (3. Ausgabe (1997), Seiten 944-946), zu bestimmen. Die Lagerungstemperatur liegt im Bereich von 0-30 °C, bevorzugt bei 2 bis 10 °C und insbesondere bevorzugt bei 2-8 °C. Auch bei Lagerungstemperaturen im Bereich der Körpertemperatur (37 °C) sind noch positive stabilisierende Effekte der Fibrinogen-Formulierung zu erwarten. Besonders bevorzugt ist eine Fibrinogen-Formulierung, die mehr als 3 und insbesondere mehr als 6 Monate stabil ist. In einer besonders bevorzugten Ausführung ist die erfindungsgemäße Fibrinogen-Formulierung 24 Monate oder darüber hinaus stabil. Die erfindungsgemäße Fibrinogen-Formulierung kann also bevorzugt eingesetzt werden, wo eine längere Lagerung im flüssigen oder viskos-flüssigen Zustand von einem Monat und mehr gewünscht ist.

Unter dem Begriff Fibrinogen ist vorzugsweise humanes Fibrinogen zu verstehen, das z.B. aus humanem Blutplasma gewonnen wurde. Dabei kann es sowohl aus gepoolten Plasmaspenden als auch aus Einzelspenden isoliert sein. Umfasst ist aber auch Fibrinogen isoliert aus Blutplasma oder der Milch von Tieren wie vorzugsweise Säugetieren (z.B. Schwein, Pferd, Rind, Ziege, Schaf und Hund). Wenn Fibrinogen aus Blutplasma isoliert und/oder gereinigt wird, kommt es meistens zur gleichzeitigen Aufreinigung weiterer Plasmaproteine, wie insbesondere Faktor XIII (F XIII). Weitere co-isolierte Plasmaproteine können z.B. je nach Reinigungsart Fibronektin, Gerinnungsfaktoren, von Willebrandfaktor, Serumalbumin und/oder Wachstumsfaktoren sein. Neben Fibrinogen als Hauptbestandteil können in der erfindungsgemäßen Fibrinogen-Formulierung also noch weitere Plasmaproteine enthalten sein. Beispielsweise handelt es sich hierbei um Faktor XIII. FXIII wird durch Thrombin in die aktivierte Form überführt und vermag die polymerisierten Fibrinmoleküle kovalent zu verbinden. Dies ist insbesondere von Vorteil, wenn die Fibrinogen-Formulierung für Fibrinkleber verwendet wird.

Der Begriff Fibrinogen, der für die erfindungsgemäße Fibrinogenformulierung verwendet wird, umfasst aber auch Fibrinogen oder aktive Fibrinogenderivate, die nach rekombinanten Methoden hergestellt wurden. Eine bevorzugte rekombinante Herstellungsmöglichkeit für humanes Fibrinogen ist aus Körperflüssigkeiten, insbesondere der Milch, von transgenen Tieren. Für bekannte Techniken sei hier beispielsweise verwiesen auf die US 5,639,940 bzw. die WO 95/23868. Rekombinant hergestelltes Fibrinogen kann in der erfindungsgemäßen Fibrinogen-Formulierung für Anwendungen wie z.B. dem Fibrinkleber noch mit weiteren Proteinen, insbesondere FXIII, versetzt sein, um die Beschaffenheit des Klebers zu verändern (siehe beispielsweise WO 99/56797) oder kann Zusätze enthalten, die für eine langsame Freisetzung bestimmt sind.

Wird das in der Fibrinogen-Formulierung verwendete Fibrinogen aus transgener/rekombinanter Herstellung, Plasma und insbesondere aus humanem Plasma isoliert, wird das verwendete Fibrinogen bevorzugt einem oder mehreren Virusinaktivierungs- bzw. Virusabreicherungsverfahren unterzogen. Dabei handelt es sich um gängige Verfahren, wie beispielsweise Pasteurisierung, Erhitzung im trockenen Zustand unter Sauerstoffausschluss, Nanofiltration, chemische Zusätze (beispielsweise Detergenzien ), UV-Bestrahlung oder Kombinationen davon.

Fibrinogen liegt in der Fibrinogen-Formulierung vorzugsweise als monomere Einheit vor, es sind aber auch multimere Einheiten möglich, die aber vorzugsweise nicht die Produkteigenschaften negativ beeinflussen. Insbesondere soll durch die Wahl der Formulierungsbestandteile und des Verhältnisses Komplexbildner zu zweiwertigen Metallionen die Bildung kovalent verknüpfter Aggregate mit mehr als fünf Fibrinogenmolekülen während der Lagerung vermieden werden.

Der Begriff "flüssig" im Sinne der vorliegenden Erfindung umfasst vorzugsweise wässrige Lösungen, also Lösungen, die notwendigerweise auch Wasser enthalten. Umfasst sind aber auch "nicht wässrige" Lösungen, wie z.B. Dimethylsulfoxid, Glycerol, Polyethylenglykol und Polypropylenglykol oder Mischungen davon. Umfasst sind auch wässrige Lösungen, die mit "nicht wässrigen" Lösungen kombiniert werden. Der Begriff "viskos-flüssig" umfasst flüssige Lösungen, die aufgrund von physikalischen oder biochemischen Veränderungen oder aufgrund von Formulierungsbestandteilen, wie beispielsweise Hyaluronsäuren, zähflüssig sind. Umfasst sind weiterhin nicht-wässrige Suspensionen, wie z.B. alkoholische Suspensionen.

Das Fibrinogen kann direkt nach der Herstellung in der erfindungsgemäßen flüssigen Fibrinogen-Formulierung gelagert werden, oder aber zwischenzeitlich als Lyophilisat oder im eingefrorenen Zustand gelagert werden. Die als Flüssigkeit vorliegende erfindungsgemäße Fibrinogen-Formulierung ist direkt am Patienten einsetzbar.

Bei den zweiwertigen Metallionen in der erfindungsgemäßen Fibrinogen-Formulierung kann es sich beispielsweise um Erdalkalimetalle, wie beispielsweise Magnesium- und Calciumionen, oder um Elemente der Nebengruppen, wie beispielsweise Zink-, oder Manganionen handeln. Insbesondere bevorzugt sind Calcium- und Zinkionen die beispielsweise in Form von CaCl₂ und ZnCl₂ zugesetzt werden können. Prinzipiell sind solche zweiwertigen Ionen geeignet, die die Stabilität von Fibrinogen erhöhen, bei Verabreichung am Patienten in der verwendeten Konzentration aber keine wesentlichen nachteiligen Nebenwirkungen verursachen. Die Konzentration der zweiwertigen Metallionen liegt im Bereich bis zu 100 mM, bevorzugt im Bereich bis zu 40 mM und insbesondere bevorzugt zwischen 0,02 und 10 mM. Die bevorzugten Konzentrationen sind in Abhängigkeit von den verwendeten zweiwertigen Ionen zu wählen. So liegt die insbesonders bevorzugte Konzentration für Calciumionen beispielsweise im Bereich von ca. 1,5 bis 10 mM, während für Zinkionen die insbesonders bevorzugte Konzentration im Bereich von 0,02 bis 1,5 mM liegt.

Ein weiterer Formulierungsbestandteil der erfindungsgemäßen Fibrinogen-Formulierung ist ein Komplexbildner. Dieser Komplexbildner ist in der Lage, zweiwertige lonen wie beispielsweise Calcium ionen zu binden. Überraschenderweise konnte im Rahmen dieser Erfindung gezeigt werden, dass durch Zusatz eines solchen Komplexbildners die Stabilität der flüssigen Fibrinogenformulierung weiter gesteigert werden kann, obwohl zu erwarten ist, dass ein wesentlicher Anteil der stabilisierenden, zweiwertigen Ionen in Anwesenheit eines Komplexbildners in komplexierter Form vorliegt. Mögliche Komplexbildner sind beispielsweise Citrat, Oxalat und EDTA (Ethylendiamintetraessigsäure). Besonders bevorzugt handelt es sich hierbei um Citrat, das beispielsweise als Natriumcitrat zugesetzt werden kann. Der Komplexbildner liegt in der erfindungsgemäßen Formulierung vorzugsweise in höherer Konzentration als die zweiwertigen Metallionen vor. Der Komplexbildner kann in Konzentrationen bis zu 150 mM vorliegen, vorzugsweise jedoch bis zu 50 mM und insbesondere vorzugsweise bis zu 25 mM.

Die erfindungsgemäße Fibrinogen-Formulierung kann weitere gängige Formulierungsbestandteile wie beispielsweise einwertige Metallsalze, Aminosäuren, Lyophilisationshilfsstoffe, Kohlenhydrate, Detergentien, chaotrope Agentien, Inhibitoren wie Fibrinolyse- oder Fibrinogenolyseinhibitoren, Proteaseinhibitoren, Plasmaproteine, Antioxidantien, Puffersubstanzen oder Mischungen davon enthalten. Diese Zusätze sind dem Fachmann weitgehend bekannt und sind aufgrund verschiedener Anforderungen auszuwählen. Zusätze können beispielsweise in Abhängigkeit von einer geplanten Zwischenlagerung (Lyophilisat, gefrorener Zustand) ausgewählt werden. Bekannte Lyophilisationshilfsstoffe für Proteine sind beispielsweise Saccharide wie Sucrose und Dextran, Zuckeralkohole wie z.B. Mannitol und Sorbitol oder Aminosäuren. Entscheidend für die jeweilige Formulierung ist natürlich auch die geplante Verwendung der Formulierung. Für eine intravenöse Verabreichung von Fibrinogen können andere dem Fachmann bekannte Zusätze angeraten sein, wie für einen Einsatz als Fibrinkleber.

Als einwertige Metallsalze sind einige dem Fachmann bekannte Alkalimetallsalze geeignet, bevorzugt sind Natrium- und Kaliumsalze bzw. Mischungen davon. Insbesondere bevorzugt sind Natriumionen, die in Form von z.B. Natriumchlorid vorliegen können. Die Konzentration der einwertigen Metallsalze liegt vorzugsweise bei ≤ 300 mM. In einer besonders bevorzugten Ausführungsform liegt die Konzentration bei ≤ 200 mM.

Der Begriff Aminosäure umfasst sowohl natürlich vorkommende Aminosäuren als auch Derivate davon. Besonders bevorzugt sind z.B. neutrale Aminosäuren wie Glycin und die sauren oder basischen Aminosäuren (Asparaginsäure, Glutaminsäure bzw. Histidin, Lysin, Arginin) oder Mischungen davon. Insbesondere bevorzugt werden Arginin oder Mischungen mit Arginin eingesetzt. Jedoch sind auch Aminosäurederivate wie beispielsweise Citrullin einsetzbar.

Zu möglichen Inhibitoren zählen Proteinaseinhibitoren, die aus gängigen Substanzen zu wählen sind, bevorzugt wird das Aprotinin oder Inhibitoren ähnlicher Spezifität verwendet.

Zu Fibrinolyseinhibitoren zählen z.B. Antiplasmine, umfassend α-2-Antiplasmin, α-2-Makroglobulin, α-1-Antiplasmin, Plasminogenaktivatorinhibitoren (PAI), umfassend PAI-1 und PAI-2, Thrombin-aktivierbarer-Fibrinolyse-Inhibitor (TAFI), Aprotinin und/oder synthetische Substanzen wie ε-Aminocapronsäure oder p-Aminomethylbenzoesäure. Fibrinolyseinhibitoren sind insbesondere bei Anwendungen interessant, wo entstehende Fibrinpolymerisate möglichst lange stabil sein sollen.

Als Fibrinogenolyseinhibitoren können beispielsweise Antiplasmine, C1-lnhibitor und Antithrombin, wobei Antithrombin bevorzugt in Gegenwart von Heparin verwendet wird, eingesetzt werden.

Bei den Plasmaproteinen kann es sich, wie bereits erwähnt, um Gerinnungsfaktoren wie beispielsweise F XIII oder um Proteine wie z.B. Fibronektin, von Willebrandfaktor, Serumalbumin oder Wachstumsfaktoren handeln. Sie können bereits als kontaminierende Proteine in dem isolierten Fibrinogen vorliegen, oder nachträglich der Fibrinogen-Formulierung zugesetzt werden. Bei den Plasmaproteinen soll es sich aber nicht um Thrombin handeln, da die gleichzeitige Lagerung von Fibrinogen und Thrombin zur Fibrinbildung führen kann und die Stabilität der Fibrinogen-Formulierung nicht zu gewährleisten ist.

Zu Kohlenhydraten zählen beispielsweise Glucose, Fructose, Saccharose, Maltose, Mannose, Trehalose, Laktose, Cellulose und Stärke oder Derivate davon. Auch in Form von Mischungen. Umfasst sind auch Zuckeralkohole wie Sorbitol und Mannitol. Unter Kohlenhydrate im Sinne der Erfindung sind auch Heteropolysaccharide zu verstehen. Hierzu zählen beispielsweise Glykosaminoglykane wie insbesondere Hyaluronsäure.

Zu Detergenzien zählen ebenfalls gängige Substanzen, bevorzugt aber nicht ionische Detergenzien wie beispielsweise Poloxamere oder Polysorbate.

Unter chaotropen Agentien sind Mittel zu verstehen, die Wasserstoffbrückenbindungen lösen. Besonders bevorzugt sind Harnstoff und Guanidin bzw. Guanidino-Gruppen-haltige Zusätze sowie verwandte Verbindungen oder Mischungen davon oder chaotrope Salze wie z.B. KJ.

Als Antioxidantien können gängige Substanzen und bevorzugt Ascorbinsäure verwendet werden.

Puffersubstanzen sind Substanzen, die den pH-Wert der Fibrinogen-Formulierung einstellen und weitgehend konstant halten. In den bevorzugten Ausführungsformen wird der pH Wert der Formulierung eingestellt auf einen Wert zwischen 5.0 und 8.0, bevorzugterweise zwischen 6.0 und 8.0, besonders bevorzugt liegt der pH-Wert zwischen 6.5 und 7.5. Bei derartigen Puffersubstanzen kann es sich beispielsweise um Aminosäuren und/oder Citrat handeln wie in einer besonders bevorzugten Ausführungsform um Citrat. Andere gängige Puffersysteme, wie beispielsweise Tris(hydroxymethyl)-aminomethan, Phosphat, Acetat, Succinat, Glycylglycin, Carbonat und Bicarbonat sind ebenfalls umfasst. Die Einstellung oder Korrektur des pH Wertes kann durch Säuren oder Basen, wie beispielsweise HCl und NaOH erfolgen.

Für alle verwendbaren Formulierungsbestandteile gilt, dass sie beim Patienten in der verwendeten Menge keine wesentlich nachteiligen Nebenwirkungen hervorrufen dürfen.

In einer besonders bevorzugten Ausführung enthält eine erfindungsgemäße Fibrinogen-Formulierung neben Calciumchlorid und Natriumcitrat z.B. noch Natriumchlorid und Arginin. In einer weiteren bevorzugten Formulierung ist zusätzlich zu Calciumchlorid, Natriumcitrat, Natriumchlorid und Arginin noch Aprotinin oder C1-Inhibitor enthalten.

Weitere insbesondere bevorzugte Fibrinogen-Formulierungen sind den Beispielen zu entnehmen.

Bei der Herstellung der erfindungsgemäßen Fibrinogen-Formulierung wird Fibrinogen zunächst nach dem Fachmann bekannten Methoden gewonnen (isoliert und/oder gereinigt). Die Gewinnung erfolgt bevorzugt aus humanem Plasma, kann aber auch aus tierischem Plasma erfolgen. Rekombinantes Fibrinogen kann z.B. aus dem Fermentationsüberstand von Animalzellkulturen oder der Milch transgener Tiere gewonnen werden. Befindet sich das gewonnene Fibrinogen zunächst in einer Lösung, die nicht der erfindungsgemäßen Fibrinogen-Formulierung entspricht, so wird diese ersetzt durch eine Lösung, die die in den Ausführungsformen beschriebenen erfindungsgemäßen Formulierungsbestandteile, insbesondere zweiwertige Metallionen, enthält. Ein Austausch oder Angleichen verschiedener Lösungen ist z.B. möglich durch Methoden wie die Ultrafiltration, Dialyse, Verdünnungen oder Zusatz von fehlenden Formulierungsbestandteilen. Sollte Fibrinogen nach bekannten Fällungsmethoden (z.B. mit Ethanol, Polyethylenglykol, Ammoniumsulfat, Glycin) ausgefällt vorliegen, kann es in einer Lösung mit den erfindungsgemäßen zweiwertigen lonen und weiteren Formulierungsbestandteilen resuspendiert oder gelöst werden. Liegt Fibrinogen als Lyophilisat vor, kann es in einer Lösung rekonstituiert werden, so dass die entstehende Formulierung der erfindungsgemäßen Fibrinogen-Formulierung entspricht.

Die so erhaltene erfindungsgemäße Fibrinogen-Formulierung kann direkt im flüssigen Zustand gelagert werden. Alternativ kann die flüssige Formulierung zwischenzeitlich auch eingefroren oder lyophilisiert werden und nach dem Auftauen oder Rekonstituieren im flüssigen Zustand entsprechend einer erfindungsgemäßen Fibrinogen-Formulierung weitergelagert werden.

Eine besonders bevorzugte Herstellung der erfindungsgemäßen Fibrinogen-Formulierung erfolgt mit Fibrinogen, das aus humanem Blutplasma gewonnen wird und die folgenden Hauptschritte umfasst:
- Herstellung einer Plasma-Rohfraktion
- Adsorption an Aluminiumhydroxid
- Virusinaktivierung
- Fällung
- Weitere Reinigungs- und/oder Virus-lnaktivierungsschritte
- Ersatz von Lösungskomponenten durch eine Lösung enthaltend mindestens ein zweiwertiges Metallsalz sowie weitere Formulierungsbestandteile, pH-Einstellung und Konzentrationseinstellung durch Ultrafiltration, Dialyse und/oder Verdünnung
- Sterilfiltration
- Lagerung der Fibrinogen-Formulierung direkt im flüssigen Zustand oder zwischenzeitlich eingefroren bzw. lyophilisiert mit anschließender Lagerung im flüssigen Zustand durch Auftauen oder Rekonstituieren.

Ein weiterer Gegenstand der Erfindung ist die Verwendung der erfindungsgemäßen Fibrinogen-Formulierung. Mögliche Anwendungen sind dem Fachmann bekannt und die erfindungsgemäße Fibrinogen-Formulierung kann für alle bekannten Verwendungen von Fibrinogen eingesetzt werden. Generell ist die erfindungsgemäße Fibrinogen-Formulierung für die Therapie von Fibrinogenmangelzuständen geeignet. Zu diesen Mangelzuständen kann es beispielsweise bei großen Wunden und nach starken Blutungen, bei großflächigen Verbrennungen, krankhafter Aktivierung der Blutstillung (Verbrauchskoagulopathie auch DIC (disseminated intravascular coagulation) genannt), durch Medikamente oder schwere Lebererkrankungen (z.B. bei Synthesestörung durch Leberparenchymschaden) kommen. Neben den beschriebenen erworbenen Hypofibrinogenämien (vermindertes Fibrinogen im Blut) bzw. Afibrinogenämien (fehlendes oder hochgradig vermindertes Fibrinogen im Blut) gibt es in seltenen Fällen auch eine angeborene Afibrinogenämie bzw. Hypofibrinogenämie , die durch fehlende oder verminderte Fibrinogensynthese in der Leber verursacht sein kann.

Bei Hypofibrinogenämien bzw. Afibrinogenämien wird die erfindungsgemäße Fibrinogen-Formulierung dem Patienten vorzugsweise intravenös injiziert, um entsprechende Mangelzustände an Fibrinogen zu kompensieren. Dosierungen orientieren sich an der Höhe des auftretenden Mangels.

Eine hohe Bedeutung kommt Fibrinogen bei der Fibrintherapie, als wichtige Komponente so genannter Fibrinkleber, zu. Ein Fibrinkleber simuliert den letzten Schritt der Blutgerinnung, indem es bei Kombination von Fibrinogen mit Thrombin in Anwesenheit von Calcium und FXIII zur Bildung von vernetztem Fibrin kommt.

In der Medizin gibt es vielfältige Anwendungsmöglichkeiten für Fibrinkleber. Wichtig zu nennen sind die Blutstillung, der Wundverschluß, die Adhäsionsprophylaxe und die Wundheilung. Die örtliche intraoperative Blutstillung ist insbesondere an parenchymatösen Organen sowie im kardiovaskulären Bereich von Bedeutung. Selbst starke Blutungen nach schweren Leberverletzungen sind so zu stillen. Auch für den Verschluß und die Fixierung von Hautwunden (einschließlich Hauttransplantat), für die Abdichtung von Nähten (z.B. am Duodenalstumpf) werden Fibrinkleber eingesetzt. Beispielhaft genannt sei auch die Verwendung beim plastischen Duraersatz und zur Hohlraumverödung sowie zur Verklebung der Pleurablätter zur Palliativbehandlung von Pleuraergüssen. Zum Kleben können die Fibrinkleber auch vorteilhaft bei Bindegeweben wie Knochen, Knorpel und Sehnen eingesetzt werden.

Fibrinogen kann auch als eine Komponente zur Herstellung einer Fibrin-Matrix verwendet werden. Ein solches Trägermaterial kann zur langsamen Freisetzung von Wirkstoffen, wie beispielsweise Wachstumsfaktoren (z.B. mit osteoinduktiven Proteinen als Matrix für Knochenregeneration), Antibiotika, entzündungshemmenden, cytostatischen oder wundheilungsfördernden Zusätzen, verwendet werden. Der Träger kann auch aus einer Mischung von Fibrin mit anderen Materialien bestehen.

Eine Fibrin-Matrix findet darüber hinaus weitreichende Verwendungsmöglichkeiten in der Biotechnologie, wie beispielsweise als Trägermaterial und Kulturmedium für Zellen und Gewebe im Tissue Engineering oder zum Umhüllen von Implantaten wie beispielsweise Biosensoren.

Weiterhin soll die Erfindung durch die folgenden Beispiele veranschaulicht werden, die jedoch in keiner Weise einschränkende Wirkung haben sollen.

### Beispiel 1:

### Herstellung von Fibrinogen aus humanem Blutplasma

Für die Gewinnung von Fibrinogen-Ausgangsmaterial wurde Kryopräzipitat in NaCl/Glycin-Lösung gelöst und eine Adsorption mit 10% (v/v) Aluminiumhydroxid Suspension (Al(OH)₃) durchgeführt. Nachdem das Al(OH)₃ abzentrifugiert worden war, wurde der verbleibende Überstand mit Glycin (Endkonzentration 2,45 M) ausgefällt.
Für die Weiterverarbeitung wurde das fibrinogenreiche Präzipitat in NaCI-Lösung gelöst und der pH-Wert der Lösung auf 7,3 eingestellt. Die Fibrinogenlösung wurde mit 80 mL Aluminiumhydroxid-Suspension pro Liter Lösung adsorbiert. Anschließend wurde das Al(OH)₃ mittels Filtration oder Zentrifugation abgetrennt und verworfen.
Für die anschließende Pasteurisierung wurde die Fibrinogenlösung mit physiologischer NaCI-Lösung auf OD₂₈₀₋₃₂₀ₙₘ = 48 verdünnt. Der Lösung wurden unter Rühren pro Liter Lösung 0,37 g Calciumchlorid-Dihydrat, 1000 g Saccharose und 75 g Glycin hinzugegeben. Der pH-Wert wurde auf pH 7,5 gehalten.

Anschließend wurde die Lösung auf +60°C erhitzt und die Temperatur für 10 h konstant gehalten. Danach wurde die Lösung abgekühlt.

Die nun pasteurisierte Fibrinogenlösung wurde mit dem dreifachen Volumen Verdünnungslösung (3,5 g/L NaCl; 6 g/L tri-Natriumcitrat-dihydrat in Wasser) versetzt. Pro Liter verdünnter Lösung wurden 90 g Glycin unter Rühren zugesetzt. Das entstandene Präzipitat wurde mittels Zentrifugation oder Filtration abgetrennt und verworfen.

Dem Überstand wurden pro Liter weitere 75 g Glycin zugesetzt. Das fibrinogenreiche Präzipitat wurde mittels Zentrifugation gewonnen und bis zur weiteren Verarbeitung bei -25 °C gelagert.

Für die weitere Reinigung und Plasminogenentfernung wurde das fibrinogenreiche Präzipitat zunächst in einem geeigneten wässrigen Lösungmittel (50 mM NaCI; 20 mM tri-Natriumcitrat-dihydrat) gelöst und vorzugsweise nach Dialyse gegen das Lösungsmittel, über eine Chromatographiesäule mit einer Matrix, die als Liganden L-Lysyl Reste trägt, gepumpt.

Zur Herstellung von Fibrinogen-Formulierungen wurde die fibrinogenhaltige Lösung zunächst mittels geeigneter Ultrafiltrationsverfahren je nach Verwendung auf eine Proteinkonzentration von ca. OD₂₈₀₋₃₂₀ₙₘ = 2 - 200, vorzugsweise ca. 20 - 160, gebracht und anschließend gegen Lösungen, die die im Beispiel 2 genannten Formulierungsbestandteile enthielten, dialysiert.

Nach Sterilfiltration erhielt man Fibrinogen-Formulierungen, die, entsprechend den nachfolgenden Beispielen, auf Stabilität getestet wurden.

### Beispiel 2:

### Stabilitätsuntersuchungen an Fibrinogen-Formulierungen

Zur Untersuchung der Stabilität von Fibrinogen in unterschiedlichen Formulierungen, wurde humanes Fibrinogen, hergestellt wie in Beispiel 1 beschrieben, verwendet. Dazu wurden Behälter mit Fibrinogen-Lösungen (OD₂₈₀₋₃₂₀ₙₘ = ca. 100) in verschiedenen Formulierungen für unterschiedliche Lagerungszeiträume (0, 1, 2, 3, und/oder mehr Monaten) bei entweder 30°C oder 2-8°C gelagert. Nach den entsprechenden Lagerungszeiträumen wurde der verbleibende Gehalt von Fibrinogen bestimmt. Dazu wurde der Anteil an gerinnbarem Protein, wie in der Fibrinkleber Monographie des Europäischen Arzneibuches (3. Ausgabe (1997), Seiten 944-946) beschrieben, bestimmt.

In den drei nachfolgend aufgeführten Formulierungen wurden neben 200 mM NaCI und 10% L-Arg x HCI bei einem pH von 7,2 entweder Natriumcitrat, oder Calciumchlorid oder Natriumcitrat kombiniert mit Calciumchlorid zugesetzt.
Formulierung 1: 20 mM Na₃-Citrat / 200 mM NaCI /10% L-Arg x HCI / pH 7,2
Formulierung 2: 2,5 mM CaCl₂/ 200 mM NaCI /10% L-Arg x HCI / pH 7,2
Formulierung 3: 20 mM Na₃-Citrat / 2,5 mM CaCl₂/ 200 mM NaCI /10% L-Arg x HCI / pH 7,2

**Tabelle 1:Gerinnbares Protein, Lagerung bei 30°C**

| | Lagerzeit | | | |
|---|---|---|---|---|
| | 0 | 1 Monat | 2 Monate | 3 Monate |
| Formulierung 1 | 100% | 93,1 % | 46,1 % | 28,9 % |
| Formulierung 2 | 100% | 93,5% | 79,6% | 61,9% |
| Formulierung 3 | 100% | 99,7 % | 101,6% | 92,6% |

**Tabelle 2: Gerinnbares Protein, Lagerung bei 2-8°C**

| | Lagerzeit | | | |
|---|---|---|---|---|
| | 0 | 1 Monat | 3 Monate | 6 Monate |
| Formulierung 1 | 100% | 110,8% | 99,4 % | 91,6% |
| Formulierung 3 | 100% | 110,9% | 106,2% | 97,5% |

Die Versuche zeigen eindeutig, dass durch Zusatz von CaCl₂ (Formulierung 2) eine deutliche Stabilisierung des Fibrinogens bei Lagerung von 30°C gegenüber der Kontrollformulierung ohne CaCl₂ (Formulierung 1) erreicht werden kann. Selbst nach dreimonatiger Lagerung ist noch mehr als doppelt soviel gerinnbares Fibrinogen nachweisbar, wie in der Kontrollformulierung (Tabelle1). Noch deutlicher wird die Stabilitätssteigerung in der Formulierung, die CaCl₂ mit dem Komplexbildner Natriumcitrat kombiniert (Formulierung 3) enthält, die nach dreimonatiger Lagerung bei 30°C sogar noch ungefähr dreimal soviel gerinnbares Protein enthält wie die Kontrollformulierung 1. Weit weniger deutlich, aber immer noch feststellbar, ist der stabilisierende Effekt von CaCl₂ und Natriumcitrat (Formulierung 3) gegenüber der Kontrollformulierung bei einer Lagerung von 3 oder 6 Monaten bei 2-8°C (Tabelle 2). Da generell bei einer Lagerung bei 2-8°C mit einer besseren Stabilität zu rechnen ist, kann der Unterschied erwartungsgemäß nur geringer ausfallen.

In weiteren Versuchen wurden verschiedene Formulierungen untersucht, in denen die Konzentrationen der übrigen Formulierungsbestandteile verringert wurden. Die verwendeten Fibrinogen-Formulierungen (OD₂₈₀₋₃₂₀ₙₘ= ca. 134 bis 146) wurden vergleichbar wie in Beispiel 1 beschrieben hergestellt und wurden für unterschiedliche Lagerungszeiträume (0, 1, 2 und/oder 3 Monate) bei 30 °C gelagert. Nach den entsprechenden Lagerungszeiträumen wurde entweder der Anteil an gerinnbarem Protein (Fibrinkleber Monographie des Europäischen Arzneibuches, 3. Ausgabe 1997, Seiten 944-946) oder Fibrinogen nach Clauss (Clauss (1957), Acta-Haematol. 17, 237-246) bestimmt (Tabelle 3 und 4).
Formulierung 4: 4 mM Na₃-Citrat /100 mM NaCI / 5% L-Arg x HCI / pH 7,2
Formulierung 5: 4 mM Na₃-Citrat / 0,5 mM CaCl₂ / 100 mM NaCI / 5% L-Arg x HCI / pH 7,2
Formulierung 6: 20 mM Na₃-Citrat / 2,5 mM CaCl₂ / 100 mM NaCI / 5% L-Arg x HCI / pH 7,2
Formulierung 7: 12 mM Na₃-Citrat / 1,5 mM CaCl₂/ 100 mM NaCl / 5% L-Arg x HCI / pH 7,2

**Tabelle 3: Gerinnbares Protein, Lagerung bei 30°C**

| | Lagerzeit | | | |
|---|---|---|---|---|
| | 0 | 1 Monat | 2 Monate | 3 Monate |
| Formulierung 4 | 100% | 104,6 % | 73,9 % | 46,7 % |
| Formulierung 5 | 100% | 112,5 % | 95,5 % | 91,9% |

**Tabelle 4:Fibrinogen nach Clauss, Lagerung bei 30°C**

| | Lagerzeit | |
|---|---|---|
| | 0 | 1 Monat |
| Formulierung 4 | 100% | 50,2 % |
| Formulierung 5 | 100% | 70,5 % |
| Formulierung 6 | 100% | 79,5% |
| Formulierung 7 | 100% | 78,0% |

Dieser Versuch zeigt, dass auch in den Formulierungen 5 bis 7 ein deutlicher Stabilitätszuwachs gegenüber der Kontrollformulierung (Formulierung 4) zu erreichen ist, sofern Calciumchlorid bzw. Calciumchlorid und Citrat enthalten sind. Dabei ist entsprechend der Fibrinogen-Testung nach Clauss der Effekt für die höheren Konzentrationen an Calciumchlorid und Citrat tendenziell noch besser.

## Patentansprüche

1. Lagerungsstabile, flüssige oder viskos-flüssige Fibrinogen-Formulierung, **dadurch gekennzeichnet, dass** neben Fibrinogen zweiwertige Metallionen in einer Konzentration von bis zu 100 mM und ein Komplexbildner enthalten sind und die Fibrinogen-Formulierung für mindestens 1 Monat bei Lagerungstemperaturen zwischen 0 und 30 °C stabil ist.

2. Fibrinogen-Formulierung nach Anspruch 1, **dadurch gekennzeichnet, dass** es sich bei den zweiwertigen Metallionen um Calciumionen, Zinkionen oder Mischungen von beiden handelt.

3. Fibrinogen-Formulierung nach Anspruch 1 oder 2, **dadurch gekennzeichnet, dass** die zweiwertigen Metallionen in einer Konzentration bis zu 40 mM und insbesondere bevorzugt zwischen 0,02 und 10 mM vorliegen.

4. Fibrinogen-Formulierung nach Anspruch 1, **dadurch gekennzeichnet, dass** es sich bei dem Komplexbildner um Citrat handelt.

5. Fibrinogen-Formulierung nach Anspruch 1 oder 4, **dadurch gekennzeichnet, dass** der Komplexbildner in einer höheren Konzentration als die zweiwertigen Metallionen vorliegt.

6. Fibrinogen-Formulierung nach einem der Ansprüche 1 oder 4 oder 5, **dadurch gekennzeichnet, dass** der Komplexbildner in Konzentrationen bis zu 150 mM vorliegt, vorzugsweise jedoch bis zu 50 mM und insbesondere vorzugsweise bis zu 20 mM.

7. Fibrinogen-Formulierung nach einem der Ansprüche 1 bis 6, **dadurch gekennzeichnet, dass** sie einen oder mehrere weitere Formulierungsbestandteile ausgewählt aus einwertigen Metallsalzen, Aminosäuren, Lyophilisationshilfsstoffen, Detergentien, Fibrinolyseinhibitoren, Fibrinogenolyseinhibitoren, Proteaseinhibitoren, Plasmaproteinen, Kohlenhydraten, Antioxidantien, Puffersubstanzen und/oder chaotrope Agentien oder Mischungen enthält.

8. Fibrinogen-Formulierung entsprechend Anspruch 7, **dadurch gekennzeichnet, dass** es sich bei den Plasmaproteinen um Faktor XIII, Fibronektin, Serumalbumin, von Willebrandfaktor und/oder Wachstumsfaktoren handelt.

9. Fibrinogen-Formulierung nach den Ansprüchen 7 oder 8, **dadurch gekennzeichnet, dass** sie Calciumchlorid, Natriumcitrat, Natriumchlorid und Arginin enthält.

10. Fibrinogen-Formulierung nach den Ansprüchen 7 oder 8, **dadurch gekennzeichnet, dass** sie Calciumchlorid, Natriumcitrat, Natriumchlorid, Arginin und Aprotinin oder C1-Inhibitor enthält.

11. Fibrinogen-Formulierung nach den Ansprüchen 7 oder 8, **dadurch gekennzeichnet, dass** sie Calciumchlorid, Natriumcitrat, Natriumchlorid, Arginin und Albumin enthält.

12. Fibrinogen-Formulierung nach einem der Ansprüche 1 bis 11, **dadurch gekennzeichnet, dass** sie einen pH-Wert von 5.0 - 8.0 aufweist.

13. Fibrinogen-Formulierung nach Anspruch 12, **dadurch gekennzeichnet, dass** sie einen pH-Wert von 6.5 bis 7.5 aufweist.

14. Fibrinogen-Formulierung nach einem der Ansprüche 1 bis 13, **dadurch gekennzeichnet, dass** die Fibrinogen-Formulierung bei 0-30°C, bevorzugt bei 2 bis 8°C, über einen Zeitraum von mindestens 3 Monaten stabil ist.

15. Fibrinogen-Fotmulierung nach Anspruch 14, **dadurch gekennzeichnet, dass** die Fibrinogen-Formulierung bei 0 bis 30°C, bevorzugt bei 2 bis 8°C, über einen Zeitraum von mindestens 6 Monaten stabil ist

16. Fibrinogen-Formulierung nach Anspruch 14, **dadurch gekennzeichnet, dass** die Fibrinogen-Formulierung bei 0 bis 30°C, bevorzugt bei 2 bis 8°C, über einen Zeitraum von mindestens 24 Monaten stabil ist.

17. Fibrinogen-Formulierung nach einem der Ansprüche 1 bis 16, **dadurch gekennzeichnet, dass** das verwendete Fibrinogen einem oder mehreren Virusinaktivierungs- bzw. Virusabreicherungsverfahren unterzogen wurde.

18. Fibrinogen-Formulierung nach einem der Ansprüche 1 bis 16, **dadurch gekennzeichnet, dass** rekombinantes Fibrinogen verwendet wird.

19. Fibrinogen-Formulierung nach einem der Ansprüche 1 bis 16, **dadurch gekennzeichnet, dass** das Fibrinogen aus Blutplasma isoliert wurde.

20. Fibrinogen-Formulierung nach einem der Ansprüche 1 bis 19, **dadurch gekennzeichnet, dass** sie einen pH-Wert von 6.0 - 8.0 aufweist.

21. Verwendung einer Fibrinogen-Formulierung nach einem der Ansprüche 1 bis 19 als Präparat zur Behandlung von Fibrinogen Mangelzuständen.

22. Verwendung einer Fibrinogen-Formulierung nach einem der Ansprüche 1 bis 19 als Komponente eines Fibrinklebers.

23. Verwendung einer Fibrinogen-Formulierung nach einem der Ansprüche 1 bis 19 als Komponente zur Herstellung einer Fibrin-Matrix.

24. Verwendung einer Fibririogen-Formulierung nach einem der Ansprüche 1 bis 19 als Diagnostikum.

## Claims

1. Storage-stable, liquid or viscous liquid fibrinogen formulation, **characterized in that**, besides fibrinogen, divalent metal ions in a concentration of up to 100 mM and a complexing agent are present, and the fibrinogen formulation is stable at storage temperatures between 0 and 30°C for at least 1 month.

2. Fibrinogen formulation according to Claim 1, **characterized in that** the divalent metal ions are calcium ions, zinc ions or mixtures of the two.

3. Fibrinogen formulation according to Claim 1 or 2, **characterized in that** the divalent metal ions are present in a concentration of up to 40 mM and particularly preferably between 0.02 and 10 mM.

4. Fibrinogen formulation according to Claim 1, **characterized in that** the complexing agent is citrate.

5. Fibrinogen formulation according to Claim 1 or 4, **characterized in that** the complexing agent is present in a higher concentration than the divalent metal ions.

6. Fibrinogen formulation according to any of Claims 1 or 4 or 5, **characterized in that** the complexing agent is present in concentrations of up to 150 mM, but preferably up to 50 mM and particularly preferably up to 20 mM.

7. Fibrinogen formulation according to any of Claims 1 to 6, **characterized in that** it comprises one or more further formulation ingredients selected from monovalent metal salts, amino acids, lyophilization auxiliaries, detergents, fibrinolysis inhibitors, fibrinogenolysis inhibitors, protease inhibitors, plasma proteins, carbohydrates, antioxidants, buffer substances and/or chaotropic agents or mixtures.

8. Fibrinogen formulation corresponding to Claim 7, **characterized in that** the plasma proteins comprise factor XIII, fibronectin, serum albumin, von Willebrand factor and/or growth factors.

9. Fibrinogen formulation according to Claims 7 or 8, **characterized in that** it comprises calcium chloride, sodium citrate, sodium chloride and arginine.

10. Fibrinogen formulation according to Claims 7 or 8, **characterized in that** it comprises calcium chloride, sodium citrate, sodium chloride, arginine and aprotinin or C1 inhibitor.

11. Fibrinogen formulation according to Claims 7 or 8, **characterized in that** it comprises calcium chloride, sodium citrate, sodium chloride, arginine and albumin.

12. Fibrinogen formulation according to any of Claims 1 to 11, **characterized in that** it has a pH of 5.0-8.0.

13. Fibrinogen formulation according to Claim 12,
**characterized in that** it has a pH of from 6.5 to 7.5.

14. Fibrinogen formulation according to any of Claims 1 to 13, **characterized in that** the fibrinogen formulation is stable at 0-30°C, preferably at 2 to 8°C, over a period of at least 3 months.

15. Fibrinogen formulation according to Claim 14, **characterized in that** the fibrinogen formulation is stable at 0 to 30°C, preferably at 2 to 8°C, over a period of at least 6 months.

16. Fibrinogen formulation according to Claim 14, **characterized in that** the fibrinogen formulation is stable at 0 to 30°C, preferably at 2 to 8°C, over a period of at least 24 months.

17. Fibrinogen formulation according to any of Claims 1 to 16, **characterized in that** the fibrinogen used has been subjected to one or more virus inactivation or virus reduction methods.

18. Fibrinogen formulation according to any of Claims 1 to 16, **characterized in that** recombinant fibrinogen is used.

19. Fibrinogen formulation according to any of Claims 1 to 16, **characterized in that** the fibrinogen has been isolated from plasma.

20. Fibrinogen formulation according to any of Claims 1 to 19, **characterized in that** it has a pH of 6.0-8.0.

21. Use of a fibrinogen formulation according to any of Claims 1 to 19 as product for the treatment of fibrinogen deficiency states.

22. Use of a fibrinogen formulation according to any of Claims 1 to 19 as component of a fibrin sealant.

23. Use of a fibrinogen formulation according to any of Claims 1 to 19 as component for producing a fibrin matrix.

24. Use of a fibrinogen formulation according to any of Claims 1 to 19 as diagnostic aid.

## Revendications

1. Composition de fibrinogène, liquide ou liquide-visqueuse, stable au stockage, **caractérisée en ce que**, outre du fibrinogène; sont contenus des ions métalliques divalents à une concentration allant jusqu'à 100 mM et un complexant, et la composition de fibrinogène est stable pendant au moins 1 mois à des températures de stockage comprises entre 0 et 30 °C.

2. Composition de fibrinogène selon la revendication 1, **caractérisée en ce que** les ions métalliques divalents consistent en ions calcium, ions zinc ou en mélanges des deux.

3. Composition de fibrinogène selon la revendication 1 ou 2, **caractérisée en ce que** les ions métalliques divalents sont présents à une concentration allant jusqu'à 40 mM et en particulier de préférence comprise entre 0,02 et 10 mM.

4. Composition de fibrinogène selon la revendication 1, **caractérisée en ce que** le complexant est un citrate.

5. Composition de fibrinogène selon la revendication 1 ou 4, **caractérisée en ce que** le complexant est présent à une plus forte concentration que les ions métalliques divalents.

6. Composition, de fibrinogène selon l'une quelconque des revendications 1 ou 4 ou 5, **caractérisée en ce que** le complexant est présent à des concentrations allant jusqu'à 150 mM, mais de préférence jusqu'à 50 mM et en particulier de préférence jusqu'à 20 mM.

7. Composition de fibrinogène selon l'une quelconque des revendications 1 à 6, **caractérisée en ce qu'**elle contient un ou plusieurs autres composants choisis parmi des sels métalliques monovalents, des acides aminés, des adjuvants de lyophilisation, des détergents, des inhibiteurs de fibrinolyse, des inhibiteurs de fibrinogénolyse, des inhibiteurs de protéases, des protéines plasmatiques, des glucides, des antioxydants, des substances tampon et/ou des agents chaotropes ou des mélanges de ceux-ci.

8. Composition de fibrinogène selon la revendication 7, **caractérisée en ce que** les protéines plasmatiques consistent en le facteur XIII, la fibronectine, l'albumine sérique, le facteur von Willebrand et/ou des facteurs de croissance.

9. Composition de fibrinogène selon les revendications 7 ou 8, **caractérisée en ce qu'**elle contient du chlorure de calcium, du citrate de sodium, du chlorure de sodium et de l'arginine.

10. Composition de fibrinogène selon les revendications 7 ou 8, **caractérisée en ce qu'**elle contient du chlorure de calcium, du citrate de sodium, du chlorure de sodium, de l'arginine et de l'aprotinine ou de l'inhibiteur C1.

11. Composition de fibrinogène selon les revendications 7 ou 8, **caractérisée en ce qu'**elle contient du chlorure de calcium, du citrate de sodium, du chlorure de sodium, de l'arginine et de l'albumine.

12. Composition de fibrinogène selon l'une quelconque des revendications 1 à 11, **caractérisée en ce qu'**elle présente un pH de 5,0 - 8,0.

13. Composition de fibrinogène selon la revendication 12, **caractérisée en ce qu'**elle présente un pH de 6,5 à 7,5.

14. Composition de fibrinogène selon l'une quelconque des revendications 1 à 13, **caractérisée en ce que** la composition de fibrinogène est stable pendant une durée d'au moins 3 mois à une température de 0 à 30 °C, de préférence de 2 à 8 °C.

15. Composition de fibrinogène selon la revendication 14, **caractérisée en ce que** la composition de fibrinogène est stable pendant une durée d'au moins 6 mois à une température de 0 à 30 °C, de préférence de 2 à 8 °C.

16. Composition de fibrinogène selon la revendication 14, **caractérisée en ce que** la composition de fibrinogène est stable pendant une durée d'au moins 24 mois à une température de 0 à 30 °C, de préférence de 2 à 8 °C.

17. Composition de fibrinogène selon l'une quelconque des revendications 1 à 16, **caractérisée en ce que** le fibrinogène utilisé a été soumis à un ou plusieurs processus d'inactivation virale ou d'appauvrissement en virus.

18. Composition de fibrinogène selon l'une quelconque des revendications 1 à 16, **caractérisée en ce qu'**on utilise du fibrinogène recombinant.

19. Composition de fibrinogène selon l'une quelconque des revendications 1 à 16, **caractérisée en ce que** le fibrinogène a été isolé à partir de plasma sanguin.

20. Composition de fibrinogène selon l'une quelconque des revendications 1 à 19, **caractérisée en ce qu'**elle présente un pH de 6,0 - 8,0.

21. Utilisation d'une composition de fibrinogène selon l'une quelconque des revendications 1 à 19, en tant que préparation destinée au traitement d'états de carence en fibrinogène.

22. Utilisation d'une composition de fibrinogène selon l'une quelconque des revendications 1 à 19, en tant que composant d'une colle de fibrine.

23. Utilisation d'une composition de fibrinogène selon l'une quelconque des revendications 1 à 19, en tant que composant destiné à la fabrication d'une matrice de fibrine.

24. Utilisation d'une composition de fibrinogène selon l'une quelconque des revendications 1 à 19, en tant qu'agent de diagnostic.
